# FASCICULE DE BREVET EUROPEEN

(11) **EP 1 366 748 B1**
(45) Date de publication et mention de la délivrance du brevet: **22.08.2012**
(21) Numéro de dépôt: 03291140.6
(22) Date de dépôt: 15.05.2003
(51) Int. Cl.: A61K 8/04, A61K 8/37, A61K 8/891, A61K 8/92, A61Q 5/12, A61Q 5/06, B65D 83/60

(54) **Dispositif aérosol à deux compartiments comprenant une composition de traitement capillaire et procédé de traitement capillaire**
Aerosolvorrichtung mit zwei Kammern enthaltend ein Haarbehandlungsmittel und Verfahren zur Haarbehandlung
Dual-compartment aerosol device comprising a hair treatment composition and hair treatment method

(30) Priorité: 31.05.2002 FR 0206734
(43) Date de publication de la demande: 03.12.2003
(73) Titulaire: L'Oréal, 75008 Paris (FR)
(72) Inventeur: Pataut, Francoise, 75017 Paris (FR); Le Bourhis, Francois, 93300 Aubervilliers (FR)
(74) Mandataire: Bourdeau, Françoise

(56) Documents cités:
- EP-A- 0 518 723
- EP-A- 0 972 723
- EP-A- 0 990 599
- EP-A- 1 038 799
- EP-A- 1 110 879
- FR-A- 2 785 594
- US-A- 6 131 776

## Description

La présente invention est relative à un dispositif aérosol à deux compartiments comprenant une composition de traitement capillaire et un gaz comprimé particulier, et à un procédé de traitement capillaire.

Les compositions de traitement capillaire, conditionnées sous forme de spray aérosol contiennent généralement une phase liquide comprenant, dans un milieu cosmétiquement acceptable alcoolique ou plus rarement hydroalcoolique, un agent de traitement capillaire, par exemple, un agent conférant une brillance aux cheveux et/ou un conditionnement des cheveux, et un agent propulseur qui est un gaz liquéfié sous pression réduite ou dissous dans la phase liquide.

D'autres dispositifs aérosols comprenant une telle phase liquide existent, mais ils comprennent des gaz comprimés pour assurer la propulsion de la phase liquide. Or ces dispositifs à gaz comprimé présentent l'inconvénient d'une perte de pression des gaz au cours du temps. En effet, on observe une fuite des gaz comprimés au cours du temps ou lors d'une mauvaise utilisation du récipient, c'est-à-dire lorsqu'il se retrouve la tête en bas.

Cet inconvénient entraîne une mauvaise propulsion de la phase liquide et par conséquent une mauvaise répartition de la phase liquide sur les cheveux.

EP 990 599 décrit un dispositif à eux compartiments comprenant dans le premier compartiment, la composition à nébuliser et dans le second, un propulseur. La composition du premier compartiment est une préparation capillaire, comprenant dans un milieu alcoolique, plus de 0,4 % d'un agent de brillance et de conditionnement. Le gaz propulseur présent dans le second compartiment est choisi parmi les gaz de compression comme l'azote, le gaz carbonique.

EP 972 723 décrit un dispositif à eux compartiments ayant les mêmes caractéristiques que celles énoncés pour EP 990 599.

Aucun des documents EP 990 599 ni EP 972 723 ne divulgue de dispositif aérosol contenant les agents de brillance et/ou de conditionnement choisis parmi les polyorganosiloxanes phénylés, l'huile de vaseline, le myristate d'isopropyle, le palmitate d'isopropyle et leurs mélanges avec une pression de gaz comprimé comprise entre 9 et 11 bars.

La demanderesse a fait la découverte surprenante que la séparation par voie mécanique de la phase liquide alcoolique ou hydroalcoolique, du gaz comprimé assurant la propulsion, permettait de résoudre ces problèmes de fuite et de répartition de la phase liquide sur les cheveux. En outre, l'utilisation d'un dispositif aérosol à deux compartiments avec un gaz comprimé particulier permet d'obtenir un spray doux et large qui assure une répartition améliorée de la phase liquide sur les cheveux, dérange moins la coiffure et conserve la forme donnée à celle-ci.

L'invention a donc pour objet un dispositif aérosol à deux compartiments comprenant une composition de traitement capillaire et un gaz comprimé tel que décrit ci-dessous.

Un autre objet de la présente invention consiste en un procédé de traitement capillaire.

D'autres objets, caractéristiques, aspects et avantages de l'invention apparaîtront encore plus clairement à la lecture de la description et des divers exemples qui suivent.

. Le dispositif aérosol à deux compartiments selon l'invention comprend :
(a) une composition de traitement capillaire comprenant, dans un milieu cosmétiquement acceptable alcoolique ou hydroalcoolique, au moins un agent de brillance et/ou de conditionnement des cheveux en une concentration supérieure à 0,4 % en poids par rapport au poids total de la composition, dans un premier compartiment, et
(b) un gaz comprimé choisi parmi l'air, l'azote, le gaz carbonique et leurs mélanges, dans un deuxième compartiment,
(c) l'agent de brillance et/ou de conditionnement des cheveux est choisi parmi les polyorganosiloxanes phénylés, l'huile de vaseline, le myristate d'isopropyle, le palmitate d'isopropyle et leurs mélanges,
et ledit gaz comprimé est utilisé sous une pression comprise entre 9 et 11 bars.

Par agent de brillance, on entend au sens de la présente invention tout composé susceptible de conférer aux cheveux une brillance supérieure à celle des cheveux avant traitement. Cette caractéristique de brillance peut être évaluée sensoriellement ou par des dispositifs techniques appropriés comme, par exemple, les photogoniomètres.

Par agent de conditionnement, on entend au sens de la présente invention tout composé susceptible d'améliorer au moins une des propriétés cosmétiques des cheveux, telles que leur démêlage, leur douceur, leur corps ou leur lissage.

On peut utiliser dans le cadre de la présente invention des huiles siliconées non volatiles telles que les polyorganosiloxanes phénylés tels que les phényltriméthicones, les phényltriméthylsiloxydiphénylsiloxanes, les diphénylméthyldiméthyltrisiloxanes, les diphényldiméthicones, les phényldiméthicones, les polyméthyl-phénylsiloxanes, éventuellement fluorés ;

Parmi les huiles siliconées non volatiles préférées, on peut citer , les polyméthylphénylsiloxanes, et les polyméthylphénylsiloxanes perfluoroalkylés, et leurs mélanges.

Ces huiles peuvent être des huiles de silicones comportant éventuellement des groupes alkyle ou alcoxy en bout de chaîne siliconée ou pendante.

Ledit agent de brillance et/ou de conditionnement des cheveux est de préférence présent en une quantité comprise entre 0,4 et 40 % en poids, mieux encore entre 1 et 20 % en poids par rapport au poids total de la composition de traitement capillaire, bornes incluses.

Par milieu cosmétiquement acceptable, on entend un milieu compatible avec les cheveux, mais aussi d'odeur, d'aspect et de toucher agréables.

Le milieu cosmétiquement acceptable alcoolique ou hydroalcoolique comprend au moins un alcool tel qu'un alcool en C₁₋₄, par exemple l'éthanol ou l'isopropanol.

Dans un milieu hydroalcoolique, la proportion d'eau peut varier entre 1 et 95 %, et de préférence entre 15 à 90 % du poids total de la composition aérosol.

La composition de traitement capillaire peut comprendre en outre des additifs tels que des polymères fixants non ioniques, cationiques, anioniques ou amphotères, des actifs traitants, des agents hydratants tels que le glycérol, des filtres UV, des acides, des bases, des agents plastifiants, des agents solubilisants, des agents conservateurs, des colorants, des pigments, des parfums, des vitamines, des provitamines, des agents tensioactifs non ioniques, anioniques, cationiques ou amphotères, des agents anti-corrosion, et leurs mélanges.

L'homme de métier veillera à choisir les éventuels additifs et leur quantité de manière à ce qu'ils ne nuisent pas aux propriétés des compositions de la présente invention.

Ces additifs sont notamment présents dans la composition selon l'invention en une quantité allant de 0 à 20 % en poids par rapport au poids total de la composition.

De préférence, le dispositif aérosol à deux compartiments est constitué par un bidon aérosol externe comportant une poche interne soudée hermétiquement à une valve. La composition est introduite dans la poche interne et un gaz comprimé est introduit entre la poche et le bidon à une pression suffisante pour faire sortir le produit sous forme d'un spray à travers l'orifice d'une buse. Un tel dispositif est commercialisé sous le nom EP SPRAY par la société EP-SPRAY SYSTEM SA.

Les dispositifs aérosol de l'invention peuvent être de préférence des laques pour cheveux.

La présente invention concerne également un procédé de traitement capillaire qui consiste en ce que l'on vaporise la composition de traitement capillaire contenue dans le dispositif aérosol selon l'invention, sur les cheveux mouillés ou secs, et en ce que l'on laisse sécher les cheveux avec ou sans apport extérieur de chaleur.

Les exemples suivants sont donnés à titre illustratif de la présente invention.

Dans les exemples suivants, toutes les quantités sont indiquées en pour cent en poids par rapport au poids total de la composition, sauf indication contraire.

### Exemple 1

On a préparé une composition de traitement capillaire à partir des ingrédients suivants :

| | % en poids |
|---|---|
| Phényltriméthylsiloxytrisiloxane vendu sous le nom commercial 556 Cosmetic Grade Fluid par la société DOW CORNING⁽¹⁾ | 10,00 |
| Méthoxycinnamate d'éthylhexyle vendu sous la dénomination commerciale PARSOL^{®} MCX par la société ROCHE VITAMINS | 0,05 |
| Parfum | 0,10 |
| Alcool éthylique absolu qsp | 100 |

On a introduit la composition préparée ci-dessus dans le dispositif de distribution aérosol commercialisé sous le nom EP SPRAY par la société EP SPRAY SYSTEM SA décrit ci-dessus. Une valve de référence 6001 format D6 est fixée sur un bidon aérosol classique et le diffuseur est un diffuseur à buse tourbillonnaire.

La poche est remplie avec la composition comme indiqué ci-dessus. De l'air comprimé est introduit entre la poche et le bidon.

On a vaporisé la composition sur des cheveux secs. La pulvérisation se fait sous la forme d'un spray doux.

On obtient après séchage, une chevelure très brillante sans modification de la forme initiale.

### Exemple 2

On a préparé une deuxième composition de traitement capillaire à partir des ingrédients suivants :

| | % en poids |
|---|---|
| Myristate d'isopropyle | 4,00 |
| Huile de vaseline | 4,00 |
| Méthoxycinnamate d'éthylhexyle vendu sous la dénomination commerciale PARSOL^{®} MCX par la société ROCHE VITAMINS | 0,05 |
| Parfum | 0,10 |
| Alcool éthylique absolu qsp | 100 |

On a introduit la composition préparée ci-dessus dans un dispositif de distribution aérosol commercialisé sous le nom EP SPRAY tel que décrit dans l'exemple 1.

On a vaporisé la composition sur des cheveux secs.

On obtient un résultat très proche de celui obtenu dans le cas de l'exemple 1.

## Revendications

1. Dispositif aérosol à deux compartiments, comprenant :
une composition de traitement capillaire comprenant, dans un milieu cosmétiquement acceptable alcoolique ou hydroalcoolique, au moins un agent de brillance et/ou de conditionnement des cheveux en une concentration supérieure à 0,4 % en poids par rapport au poids total de la composition, dans un premier compartiment, et
un gaz comprimé choisi parmi l'air, l'azote, le gaz carbonique et leurs mélanges, dans un deuxième compartiment,
l'agent de brillance et/ou de conditionnement des cheveux est choisi parmi les polyorganosiloxanes phénylés, l'huile de vaseline, le myristate d'isopropyle, le palmitate d'isopropyle et leurs mélanges et
la pression du gaz comprimé est comprise entre 9 et 11 bars.

2. Dispositif aérosol selon l'une quelconque des revendications précédentes, **caractérisé en ce que** l'agent de brillance et/ou de conditionnement des cheveux est présent en une quantité comprise entre 0,4 et 40 % en poids par rapport au poids total de la composition, bornes incluses.

3. Dispositif aérosol selon la revendication 2, **caractérisé en ce que** l'agent de brillance et/ou de conditionnement des cheveux est présent en une quantité comprise entre 1 et 20 % en poids par rapport au poids total de la composition, bornes incluses.

4. Dispositif selon l'une quelconque des revendications précédentes, **caractérisé en ce que** le milieu cosmétiquement acceptable alcoolique ou hydroalcoolique comprend au moins un alcool.

5. Dispositif aérosol selon la revendication 4, **caractérisé en ce que** l'alcool est un alcool en C₁₋₄.

6. Dispositif aérosol selon la revendication 5, **caractérisé en ce que** l'alcool est l'éthanol ou l'isopropanol.

7. Dispositif aérosol selon l'une quelconque des revendications précédentes, **caractérisé en ce que** la composition de traitement capillaire comprend en outre des additifs choisis parmi les polymères fixants non ioniques, cationiques, anioniques ou amphotères, des actifs traitants, des agents hydratants, des filtres UV, des acides, des bases, des agents plastifiants, des agents solubilisants, des agents conservateurs, des colorants, des pigments, des parfums, des vitamines, des provitamines, des agents tensioactifs non ioniques, anioniques, cationiques ou amphotères, des agents anti-corrosion, et leurs mélanges.

8. Dispositif aérosol selon l'une quelconque des revendications précédentes, **caractérisé en ce qu'**il constitue une laque pour cheveux.

9. Procédé de traitement cosmétique des cheveux, **caractérisé en ce que** l'on vaporise la composition de traitement capillaire contenue dans le dispositif aérosol selon l'une quelconque des revendications précédentes, sur les cheveux mouillés ou secs, et **en ce que** l'on laisse sécher les cheveux avec ou sans apport extérieur de chaleur.

## Claims

1. Two-compartment aerosol device comprising:
a hair treatment composition comprising, in an alcoholic or aqueous-alcoholic cosmetically acceptable medium, at least one hair sheen agent and/or conditioner in a concentration of greater than 0.4% by weight relative to the total weight of the composition, in a first compartment, and
a compressed gas chosen from air, nitrogen and carbon dioxide, and mixtures thereof, in a second compartment,
the hair sheen agent and/or conditioner being chosen from phenylated polyorganosiloxanes, liquid petroleum jelly, isopropyl myristate and isopropyl palmitate, and mixtures thereof, and the pressure of the compressed gas being between 9 and 11 bar.

2. Aerosol device according to the preceding claim, **characterized in that** the hair sheen agent and/or conditioner is present in an amount of between 0.4% and 40% by weight relative to the total weight of the composition, limits included.

3. Aerosol device according to Claim 2, **characterized in that** the hair sheen agent and/or conditioner is present in an amount of between 1% and 20% by weight relative to the total weight of the composition, limits included.

4. Device according to any one of the preceding claims, **characterized in that** the alcoholic or aqueous-alcoholic cosmetically acceptable medium comprises at least one alcohol.

5. Aerosol device according to Claim 4, **characterized in that** the alcohol is a C₁₋₄ alcohol.

6. Aerosol device according to Claim 5, **characterized in that** the alcohol is ethanol or isopropanol.

7. Aerosol device according to any one of the preceding claims, **characterized in that** the hair treatment composition also comprises additives chosen from nonionic, cationic, anionic or amphoteric fixing polymers, treating active agents, moisturizers, UV-screening agents, acids, bases, plasticizers, solubilizers, preserving agents, colorants, pigments, fragrances, vitamins, provitamins, nonionic, anionic, cationic or amphoteric surfactants and anticorrosion agents, and mixtures thereof.

8. Aerosol device according to any one of the preceding claims, **characterized in that** it constitutes a hair lacquer.

9. Cosmetic process for treating the hair, **characterized in that** the hair treatment composition contained in the aerosol device according to any one of the preceding claims is vaporized onto wet or dry hair, and **in that** the hair is left to dry with or without supplying external heat.

## Patentansprüche

1. Zweikammer-Aerosolvorrichtung, umfassend:
eine Haarbehandlungzusammensetzung, die in einem kosmetisch unbedenklichen alkoholischen oder wässrig-alkoholischen Medium mindestens einen Haarglanzgeber und/oder mindestens ein Haarkonditionierungsmittel in einer Konzentration von mehr als 0,4 Gew.-%, bezogen auf das Gesamtgewicht der Zusammensetzung, umfasst, in einer ersten Kammer und
ein unter Luft, Stickstoff, Kohlendioxid und Mischungen davon ausgewähltes Druckgas in einer zweiten Kammer,
wobei der Haarglanzgeber und/oder das Haarkonditionierungsmittel unter phenylierten Polyorganosiloxanen, Vaselineöl, Isopropylmyristat, Isopropylpalmitat und Mischungen davon ausgewählt ist und
der Druck des Druckgases zwischen 9 und 11 bar liegt.

2. Aerosolvorrichtung nach dem vorhergehenden Anspruch, **dadurch gekennzeichnet, dass** der Haarglanzgeber und/oder das Haarkonditionierungsmittel in einer Menge zwischen 0,4 und 40 Gew.-%, bezogen auf das Gesamtgewicht der Zusammensetzung, Grenzwerte eingeschlossen, vorliegt.

3. Aerosolvorrichtung nach Anspruch 2, **dadurch gekennzeichnet, dass** der Haarglanzgeber und/oder das Haarkonditionierungsmittel in einer Menge zwischen 1 und 20 Gew.-%, bezogen auf das Gesamtgewicht der Zusammensetzung, Grenzwerte eingeschlossen, vorliegt.

4. Vorrichtung nach einem der vorhergehenden Ansprüche, **dadurch gekennzeichnet, dass** das kosmetisch unbedenkliche alkoholische oder wässrig-alkoholische Medium mindestens einen Alkohol umfasst.

5. Aerosolvorrichtung nach Anspruch 4, **dadurch gekennzeichnet, dass** es sich bei dem Alkohol um einen C₁₋₄-Alkohol handelt.

6. Aerosolvorrichtung nach Anspruch 5, **dadurch gekennzeichnet, dass** es sich bei dem Alkohol um Ethanol oder Isopropanol handelt.

7. Aerosolvorrichtung nach einem der vorhergehenden Ansprüche, **dadurch gekennzeichnet, dass** die Haarbehandlungszusammensetzung außerdem Additive umfasst, die unter nichtionischen, kationischen, anionischen oder amphoteren fixierenden Polymeren, Behandlungswirkstoffen, Hydratisierungsmitteln, UV-Filtern, Säuren, Basen, Weichmachern, Lösungsvermittlern, Konservierungsstoffen, Farbmitteln, Pigmenten, Parfümen, Vitaminen, Provitaminen, nichtionischen, anionischen, kationischen oder amphoteren Tensiden, Korrosionsschutzmitteln und Mischungen davon ausgewählt sind.

8. Aerosolvorrichtung nach einem der vorhergehenden Ansprüche, **dadurch gekennzeichnet, dass** sie einen Haarlack darstellt.

9. Verfahren zur kosmetischen Behandlung der Haare, **dadurch gekennzeichnet, dass** man die in der Aerosolvorrichtung nach einem der vorhergehenden Ansprüche enthaltene Haarbehandlungszusammensetzung auf den feuchten oder trockenen Haaren verdampft und die Haare mit oder ohne äußere Wärmezufuhr trocknen lässt.
